# EUROPEAN PATENT APPLICATION

(11) **EP 2 881 089 A1**
(43) Date of publication of application: **10.06.2015**
(21) Application number: 13195474.5
(22) Date of filing: 03.12.2013
(51) Int. Cl.: A61F 2/915

(54) **Stent for implantation into blood vessels**

(71) Applicant: Centre de Recherche Public Henri Tudor, 1855 Luxembourg (LU)
(72) Inventor: Azaouzi, Mohamed, L-1855 Luxembourg (LU)
(74) Representative: Pronovem

(57) **Abstract**

In accordance with the invention, a stent (10) comprises radially expandable ring elements (12), which are axially aligned along a central axis (A) of the stent, and longitudinally deformable bridges (114), which axially interconnect two neighbouring ring elements at circumferentially spaced points and confer bendability to the stent. The ring elements include straight longitudinal struts (116), which are parallel to the central axis of the stent and which directly interconnect the longitudinally deformable bridges arranged on both sides of a ring element.

## Description

### Technical Field

The present invention relates to a stent for implantation into blood vessels, in particular to a vascular or a coronary stent.

### Background Art

A stent is a radially expandable, plastically deformable, open-structure tube, which can be inserted into a conduit in the body to counteract a flow constriction. Vascular stents are used to keep blood vessels open e.g. in the treatment of coronary heart disease.

Coronary heart disease is mainly related to cholesterol plaque buildup, which causes hardening and narrowing of the arteries, consequently reducing or blocking blood flow to the heart. To counteract the effects associated with this vascular disease, a stent is commonly used to keep the artery open by scaffolding the arterial wall to ensure normal blood flow is maintained. A coronary or balloon-expandable stent is a tubular, often mesh-like structure made from materials that can be plastically deformed through the inflation of a balloon, which upon deployment can undergo as much as 20%-30% plastic strain. To restore blood flow across a constricted blood vessel, the stent is mounted on a catheter containing an uninflated balloon and inserted into the diseased artery. Once the stent reaches the blocked artery, it is expanded and plastically deformed under the action of radially outward pressure created by inflation of the balloon. After the balloon is deflated the stent remains in its expanded shape, except for slight recoil caused by the elastic portion of the deformation.

Most of coronary stent designs have two fundamental constituents: radially expandable ring elements, and connecting elements, referred to as "bridges", which connect adjacent rings together. Expandable rings are typically comprised of serpentine strands. As used herein, the term serpentine is intended to encompass zigzag shapes (having sharp bends), meandering shapes (with smooth bends) as well as any hybrid forms of these.

Stent design is a major factor which determines reliability of the stent during insertion into the blocked artery and throughout the long-term in vivo service. The mechanical properties of vascular stents, such as flexibility, elastic recoil, foreshortening, scaffolding and radial strength, depend on stent design characteristics, including the geometries of the radially expandable ring elements and the bridges.

### Summary of invention

According to a first aspect of the invention, in a stent comprising radially expandable ring elements, which are axially aligned along a central axis of the stent, and longitudinally deformable bridges, which axially interconnect two neighboring ring elements at circumferentially spaced points and confer bendability to the stent, the ring elements include straight longitudinal struts, which are parallel to the central axis of the stent and which directly interconnect the longitudinally deformable bridges arranged on both sides of a ring element.

As used herein, the term "ring element" designates a structural element having the form of an annular band extending about the central axis. As will be appreciated, the straight longitudinal struts confer axial dimensional stability to the ring elements, i.e. they define the longitudinal extension of the ring elements and substantially prevent the ring elements to be expanded or foreshortened in axial direction when the stent is deployed. The longitudinal bridges, on the other hand, are shaped so as to be elastically and/or plastically deformable in axial direction, thereby guaranteeing that the stent can conform to a curved conduit. The bridges preferably comprise serpentine links able to be stretched (on the outer side of a bending), compressed (on the inner side of a bending) or serving as hinges (on the neutral plane of the bending). The structure of the bridges allows them to deform elastically when subjected to relatively low stress and plastically when higher stress is applied. It will be appreciated that the proposed structure of the stent substantially decouples radial expansion of the strent from axial deformations. Specifically,when deployed, e.g. by inflation of a balloon, a stent in accordance with the invention undergoes only little or no foreshortening in axial direction while the ring elements are expanded in radial direction. It is worthwhile noting that the stent does not exhibit substantial auxetic behaviour (i.e. substantially no axial elongation will be caused by the radial expantions.)

The framework of a stent in accordance with the present invention is preferably made of metal, preferably stainless steel (such as, e.g., of type AISI316L) or cobalt-chromium alloy. The stent is preferably cut from a circular cylindrical tube, e.g. by laser-cutting, resulting in substantially rectangular cross sections of the individual strands, struts, links, bends, etc. forming the tubular stent framework. Alternatively but less preferably, the stent may be made from cylindrical braided wire meshes, coiled strip or etched sheet. The thickness of the tubular framework, as well as the widths of the individual strands, struts, links, bends, etc. are preferably comprised in the range from 0.05 mm to 0.15 mm, more preferably in the range from 0.75 mm to 0.135 mm and still more preferably in the range from 0.09 mm to 0.12 mm. The radius of the stent in the un-expanded state Rᵢ preferably amounts to a value in the range from 0.7 mm to 2 mm, preferably in the range from 1.12 mm to 1.9 mm. The radius of the stent in the expanded state R_{f} is preferably at least 3 times higher than Rᵢ.

According to a preferred embodiment, each ring element comprises a first and a second serpentine strand, forming closed rings around the central axis and connected to each other by the straight longitudinal struts. The length (in axial direction) of each ring element is preferably above 3 mm.

Preferably the parts of the first and second serpentine strands that lie between two circumferentially adjoining straight longitudinal struts of a ring element form a folded structure, which is capable of circumferentially unfolding (and undergoing a plastic deformation) when the stent is radially expanded.

The first and second serpentine strands are preferably symmetric to each other with regard to a midplane of the ring element which is perpendicular to the central axis of the stent. Such symmetry may be preferable if a uniform expansion of the stent is desired. It should be noted, however, that the serpentine shapes of the first and second strands could be deliberated asymmetric in case a nonuniform expansion in axial direction should be desired.

According to a preferred embodiment, the first and second strands are connected to each other by further (or secondary) straight struts arranged alternating with the straight longitudinal struts around the central axis. The secondary straight struts are preferably arranged parallel to the central axis of the stent and substantially shorter than the straight longitudinal struts.

Preferably, each of the first and second serpentine strands is symmetric with respect to a plane containing both a secondary straight strut and the central axis of the stent and/or with respect to a plane containing both a straight longitudinal strut and the central axis of the stent.

Preferably, at least one of the first and second serpentine strands comprises, when the stent is radially unexpanded (i.e. in the un-deployed state of the stent):
○ a first U-shaped loop comprising a first and a second arm joined by a bend, the first and second arms extending in parallel to the straight longitudinal struts, the first U-shaped loop being connected with one end of the first arm to one end of a straight longitudinal strut;
○ a J-shaped loop comprising a bend and an arm, the J-shaped loop being connected with its bend to the second arm of the first U-shaped loop, wherein the arm of the J-shaped loop extends in parallel to the second arm of the first U-shaped loop; and
○ a second U-shaped loop comprising a first and a second arm joined by a bend, the second U-shaped loop being arranged transversally with respect to the first U-shaped loop, wherein the first arm of the second U-shaped loop is connected to the arm of the J-shaped loop by an arc, and wherein the second arm of the second U-shaped loop is connected to one end of a further straight strut.

As will be appreciated, this stent design exhibits significant improvements of the structural behavior (in terms of flexibility, foreshortening, elastic recoil and radial strength) in comparison with conventional designs.

According to a preferred embodiment, the end points of deformable bridges that are directly interconnected by straight longitudinal struts are all aligned in parallel to the central axis. In other words, the deformable bridges and the longitudinal struts interconnecting them form flexible longitudinal beam elements, which run parallel to the central axis and perpendicular to the ring elements.

Preferably, longitudinally deformable bridges are configured as serpentine links. According to a particularly preferred configuration, at least part of the longitudinally deformable bridges have the form of a Greek letter Ω (omega), the plane of symmetry of which is perpendicular to the central axis of the stent and which is connected at each side to a straight longitudinal strut. The serpentine portion of each bridge preferably has a length (in the axial direction of the stent) comprised in the range from 0.5 mm to 2 mm, more preferably in the range from 1 mm to 1.5 mm, when the stent is not bent.

### Brief description of drawings

Specific embodiments of the invention will hereinafter be described, by way of example, with reference to the attached drawings, wherein:
Fig. 1: is a flat layout view of a stent in accordance with a first preferred embodiment of the invention;
Fig. 2: is a lateral view of the stent of Fig. 1 in unexpanded state;
Fig. 3: is a lateral view of the stent of Fig. 1 in expanded state;
Fig. 4: is a close-up of a unit cell of a ring element of the stent of Fig. 1;
Fig. 5 is a flat layout view of a stent in accordance with a second preferred embodiment of the invention;
Fig. 6: is a lateral view of the stent of Fig. 5 in unexpanded state;
Fig. 7: is a lateral view of the stent of Fig. 5 in expanded state;
Fig. 8: is a close-up of a unit cell of a ring element of the stent of Fig. 5.

### Description of embodiments

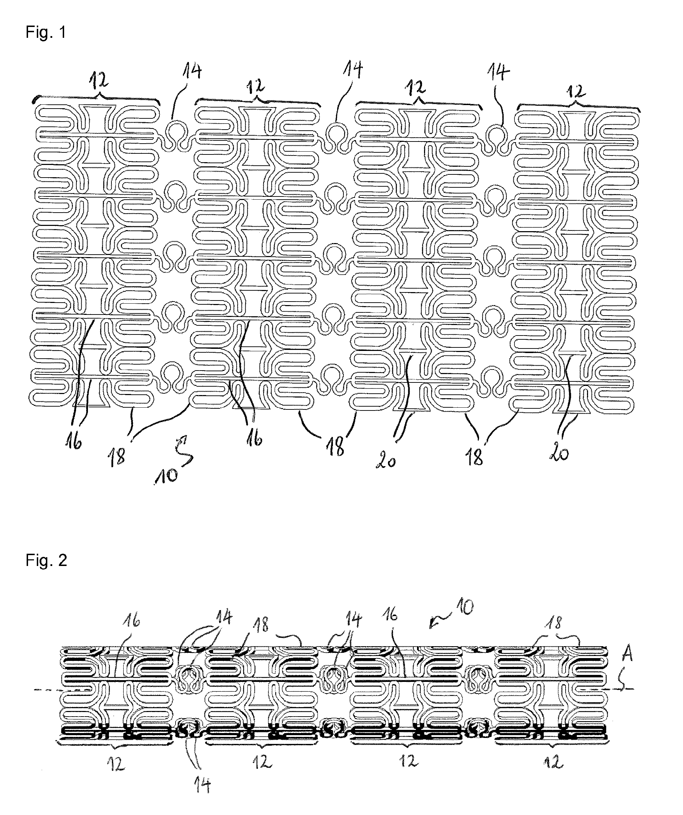

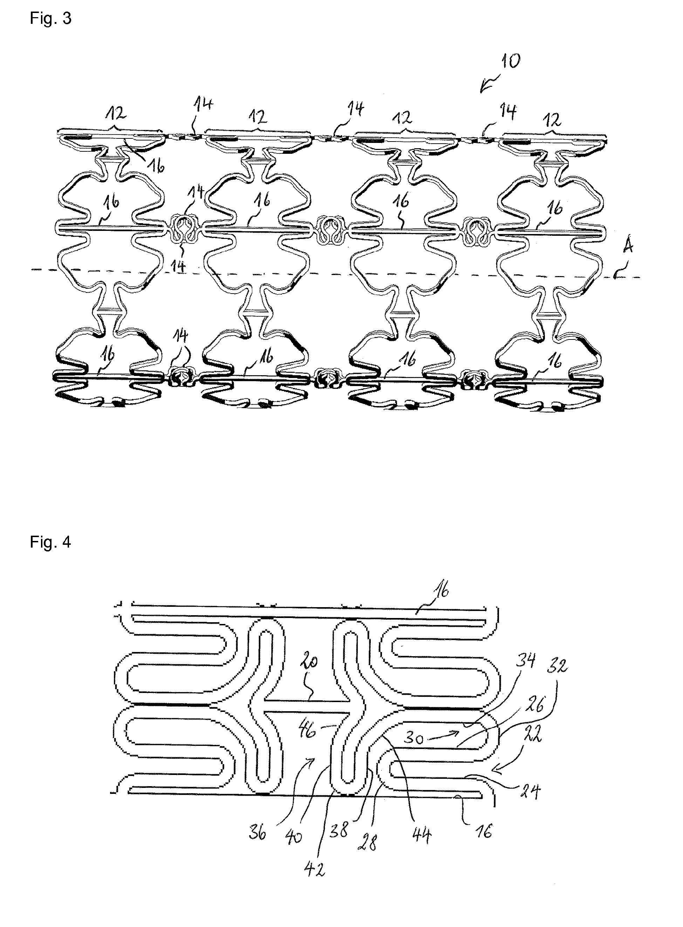

A vascular stent 10 in accordance with a first preferred embodiment of the invention is shown in and described with respect to Figs. 1 to 4. Fig. 1 shows the stent 10 in its pre-deployed state as it would appear if it were cut in parallel to the central axis and then laid out flat. Fig. 2 is the two-dimensional layout view that the stent has in its pre-deployed state, i.e. prior of being inflated by a balloon catheter and radially expanded against the wall of an artery. Fig. 3 shows the stent in its expanded state, i.e. after deployment by balloon inflation.

The illustrated stent 10 comprises several ring elements 12 arranged about the central axis A and joined to one another by bridges 14. Each ring element has an axial length of about 3 mm. In the illustrations, each pair of adjacent ring elements 12 is linked by five bridges 14 but another number of bridges could equally well be used. Each bridge 14 has a length of about 1 mm in the axial direction. While the ring elements 12 are radially deformable and render the stent radially expandable, the bridges 14 are longitudinally deformable and confer bendability to the stent (i.e. the ability of the stent to yield and deform under load applied perpendicular to the central axis.) The bendability of the stent 10 depends on the ability of the bridges 14 to lengthen, shorten and be bent. In the illustrated embodiment, the bridges are configured as serpentine links having generally the shape of an Ω (Greek capital letter omega).

The ring elements 12 comprise primary straight (longitudinal) struts 16, which run in parallel to the central axis A and which directly interconnect the bridges 14. The primary straight struts 16 confer axial dimensional stability to the ring elements 12, i.e. substantially prevent the ring elements 12 from shortening (as a whole) in axial direction.

The straight line defined by the end points of each bridge 14 is parallel to the central axis A of the stent and coincides with the primary straight struts connected by the bridge. Accordingly, the stuts and bridges that are interconnected in axial direction form flexible longitudinal beam elements, which are perpendicular to the ring elements 12. Since the connection points between these beams and the ring elements 12 are all aligned parallel to the central axis, the beams are not (or only minimally) stretched or compressed when the stent is radially expanded. Due to the structure of the stent, radial expansion of the strent is thus largely decoupled from axial deformation. This means that the illustrated strent does not substantially contract or lengthen in axial direction when expanded radially.

Each ring element comprises a first and a second serpentine strand 18 extending about the central axis A. In the un-deployed state of the stent, each serpentine strand 18 forms a folded structure, which circumferentially unfolds and undergoes plastic deformation as the stent is expanded. The first and second serpentine strands 18 of each ring element 12 are symmetric to each other with respect to the midplane of the ring element 12 which is perpendicular to the central axis A. The serpentine strands 18 of each ring element 12 are connected to each other by secondary straight struts arranged alternating with the primary straight struts 16 around the central axis. The secondary straight struts are parallel to the central axis A and substantially shorter than the primary straight struts. They connect the points on the first and second serpentine strands that are located at the greatest distance (on the strands) from the primary straight struts. Accordingly, the secondary straight struts help to control the unfolding of the strands by constraining these points with respect to each other.

Each of the first and second serpentine strands 18 of each ring element 12 is self-symmetric with respect to each plane containing both a secondary straight strut 20 and the central axis A and/or with respect to each plane containing both a primary straight strut 16 and the central axis A. It follows that each ring element 12 appears as a circumferentially repetitive pattern made of a plurality of unit cells connected to one another in circumferential direction. As best illustrated in Fig. 4, in each one of these unit cells the first and second serpentine strands 18 comprise each:
○ a first U-shaped loop 22 comprising a first 24 and a second 26 arm joined by a bend 28, the first and second arms 24, 26 extending in parallel to the straight longitudinal struts 16, the first U-shaped loop 22 being connected with one end of the first arm 24 to one end of a straight longitudinal strut;
○ a J-shaped loop 30 comprising a bend 32 and an arm 34, the J-shaped loop being connected with its bend 32 to the second arm 26 of the first U-shaped loop 22, the arm of the J-shaped loop 30 extending in parallel to the second arm 26;
○ a second U-shaped loop 36 comprising a first 38 and a second 40 arm joined by a bend 42, the second U-shaped loop being arranged transversally with respect to the first U-shaped loop 22, the first arm of the second U-shaped loop 36 being connected to the arm 34 of the J-shaped loop 30 by a first arc 44, and the second arm 40 of the second U-shaped loop 36 being connected to one end of a secondary straight strut 20 by a second arc 46; and
○ a symmetric repetition of the basic pattern containing the first U-shaped loop, the J-shaped loop and the second U-shaped loop.

As can be seen by comparing Figs. 2 and 3, the ring elements 2 undergo an important plastic deformation when the stent 10 is expanded. Due to the importance of the deformation, elastic radial recoil (RR) is small, i.e. smaller than about 5%. RR, expressed in %, is defined as RR = R_{stent-load} - R_{stent-unload})/R_{stent-load}, where R_{stent-load} is the radius of the stent with the inflated balloon catheter is place and R_{stent-unload} is the radius of the stent after removal of the balloon catheter (i.e. the relative contraction between maximal extension and final state).

Thanks to the small radial recoil and the fact that the stent does not significantly change its length during radial expansion (stent length change below 5%, preferably below 3.5%) undesirable shearing forces on the arterial wall can be reduced and the risk of injury in the form of denudation of the endothelial cells consequently be minimized.

As will further be appreciated, the stent shown in Figs. 1 to 3 provides good vessel coverage, whereby it is ensured that vessel tissue does not prolapse across the openings in the framework of the stent.

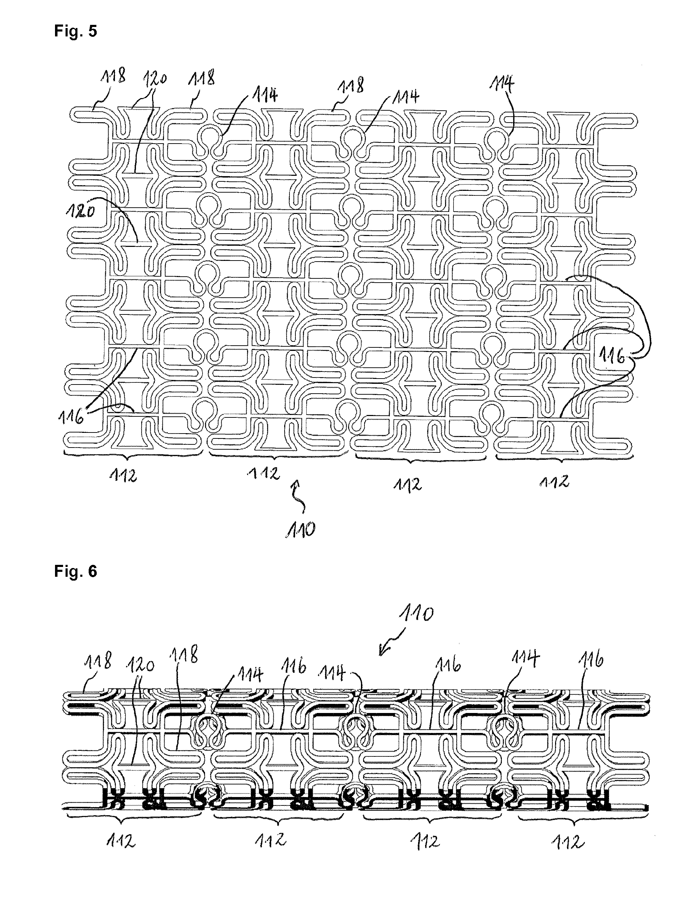

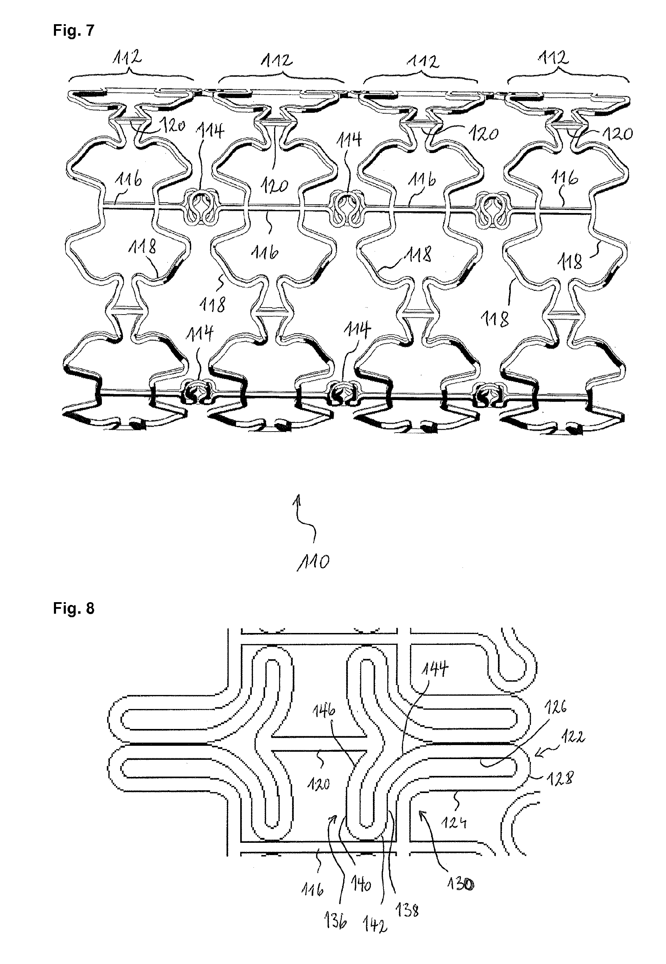

Figs. 5 to 7 illustrate a vascular stent 110 in accordance with a second preferred embodiment of the invention. Fig. 5 shows the stent 110 in its pre-deployed state as it would appear if it were cut in parallel to the central axis and then laid out flat. Fig. 6 is the two-dimensional layout view that the stent 110 has in its pre-deployed state, i.e. prior of being inflated by a balloon and radially expanded against the wall of an artery. Fig. 7 shows the stent 110 in its expanded state, i.e. after deployment by balloon inflation. The stent 110 differs from the stent 10 of Figs. 1 to 4 only in the framework layout.

The stent 110 comprises radially deformable ring elements 112 with an axial length of about 3 mm and joined to one another by axially deformable bridges 114. The bridges 114 are configured as serpentine links, having generally the shape of an Ω, connected to the ring elements 112 by straight portions on either side of the serpentine link. The serpentine portion of each bridge measures about 1 mm in axial direction.

Each one of the ring elements 112 comprises primary 116 and secondary 120 straight struts 116, which run in parallel to the central axis of the stent. The primary straight struts directly interconnect the bridges 114, thereby conferring axial dimensional stability to the longitudinal beams formed by the primary straight struts 116 and bridges 114 that are aligned in axial direction and thus to the stent 110 as a whole. The primary straight struts substantially prevent the points where the ring elements 112 are connected to the rest of the framework from moving in the axial direction when the ring elements 112 are radially expanded. As in the previously described embodiment, the structure of the stent framework is configured such that it largely decouples radial expansion from axial deformation (Poisson's ratio of the stent ≈ 0).

Each ring element 112 comprises a first and a second serpentine strand 118 extending about the central axis. In the un-deployed state of the stent, each serpentine strand 118 forms a folded structure, which circumferentially unfolds and undergoes plastic deformation as the stent 110 is expanded. The first and second serpentine strands 118 of each ring element 112 are symmetric to each other with respect to the midplane of the ring element 112 which is perpendicular to the central axis. The serpentine strands 118 of each ring element 112 are connected to each other by the secondary straight struts 120 arranged alternating with the primary straight struts 116 around the central axis. They connect the points on the first and second serpentine strands 118 that are located at the greatest distance (on the strands) from the primary straight struts 116. Accordingly, the secondary straight struts 120 help to control the unfolding of the strands 118 by constraining these points with respect to each other.

Each of the first and second serpentine strands 118 of each ring element 112 is self-symmetric with respect to each plane containing both a secondary straight strut and the central axis of the stent 110 and/or with respect to each plane containing both a primary straight strut and the central axis of the stent 110. Each ring element 12 thus appears as a circumferentially repetitive pattern made of a plurality of unit cells connected to one another in circumferential direction. As best illustrated in Fig. 8, in each one of these unit cells the first and second serpentine strands 118 comprise each :
○ a first U-shaped loop 122 comprising a first 124 and a second 126 arm joined by a bend 128, the first and second arms 124, 126 extending in parallel to the straight longitudinal struts 116, the first U-shaped loop 122 being connected with a primary straight strut 116 via a J-shaped strut 130;
○ a second U-shaped loop 136 comprising a first 138 and a second 140 arm joined by a bend 142, the second U-shaped loop being arranged transversally with respect to the first U-shaped loop 122, the first arm 138 of the second U-shaped loop 136 being connected to the second arm 126 of the first U-shaped loop 122 by a first arc 144, and the second arm 140 of the second U-shaped loop 136 being connected to one end of a secondary straight strut 120 by a second arc 146; and
○ a symmetric repetition of the basic pattern containing the first and second U-shaped loops and the J-shaped strut.

While specific embodiments have been described in detail, those skilled in the art will appreciate that various modifications and alternatives to those details could be developed in light of the overall teachings of the disclosure. Accordingly, the particular arrangements disclosed are meant to be illustrative only and not limiting as to the scope of the invention, which is to be given the full breadth of the appended claims and any and all equivalents thereof.

### Reference sign list

| | **Figs. 1-4** | | **Figs. 5-8** |
|---|---|---|---|
| 10 | Stent | 110 | stent |
| 12 | Ring element | 112 | Ring element |
| 14 | Bridge | 114 | Bridge |
| 16 | Primary straight strut | 116 | Primary straight strut |
| 18 | Serpentine strand | 118 | Serpentine strand |
| 20 | Secondary straight strut | 120 | Secondary straight strut |
| 22 | First U-shaped loop | 122 | First U-shaped loop |
| 24 | First arm | 124 | First arm |
| 26 | Second arm | 126 | Second arm |
| 28 | Bend | 128 | Bend |
| 30 | J-shaped loop | 130 | J-shaped strut |
| 32 | Bend | 136 | Second U-shaped loop |
| 34 | Arm | 138 | First arm |
| 36 | Second U-shaped loop | 140 | Second arm |
| 38 | First arm | 142 | Bend |
| 40 | Second arm | 144 | First arc |
| 42 | Bend | 146 | Second arc |
| 44 | First arc | | |
| 46 | Second arc | | |

## Claims

1. A stent comprising:
radially expandable ring elements, which are axially aligned along a central axis of the stent; and
longitudinally deformable bridges axially interconnecting two neighbouring ring elements at circumferentially spaced points, conferring bendability to the stent;
**characterized in that**
the ring elements comprise straight longitudinal struts, which are parallel to the central axis of the stent and which directly interconnect the longitudinally deformable bridges arranged on both sides of a ring element.

2. The stent as claimed in claim 1, wherein
each ring element comprises a first serpentine strand forming a closed ring around said central axis and a second serpentine strand forming a closed ring around said central axis, said first and second strands being connected to each other by said straight longitudinal struts.

3. The stent as claimed in claim 2, wherein the parts of said first and second serpentine strands arranged between two circumferentially adjoining straight longitudinal struts of a ring element form a folded structure, which is capable of circumferentially unfolding when the stent is radially expanded.

4. The device as claimed in any one of claims1 to 3, wherein the first and second serpentine strands are symmetric to each other with regard to a midplane of the ring element which is perpendicular to the central axis of the stent.

5. The stent as claimed in any one of claims 1 to 4, wherein
said first and second strands are connected to each other by further straight struts arranged alternating with said straight longitudinal struts around said central axis.

6. The device as claimed in claim 5, wherein said further straight struts are parallel to the central axis of the stent and substantially shorter than the straight longitudinal struts.

7. The device as claimed in claim 6, wherein each of the first and second serpentine strands is symmetric with regard to a plane containing both a further straight strut and the central axis of the stent and/or with regard to a plane containing both a straight longitudinal strut and the central axis of the stent.

8. The device as claimed in claim 7, wherein the at least one of said first and second serpentine strands comprises, when the stent is radially unexpanded:
a first U-shaped loop comprising a first and a second arm joined by a bend, said first and second arms extending in parallel to said straight longitudinal struts, said first U-shaped loop being connected with one end of said first arm to one end of a straight longitudinal strut;
a J-shaped loop comprising a bend and an arm, said J-shaped loop being connected with its bend to the second arm of the first U-shaped loop, wherein the arm of said J-shaped loop extends in parallel to the second arm of the first U-shaped loop; and
a second U-shaped loop comprising a first and a second arm joined by a bend, said second U-shaped loop being arranged transversally with respect to said first U-shaped loop, wherein the first arm of said second U-shaped loop is connected to the arm of said J-shaped loop by an arc, and wherein the second arm of the second U-shaped loop is connected to one end of a further straight strut.

9. The stent as claimed in any one of claims 1 to 8, wherein said straight longitudinal struts confer axial dimensional stability to said ring elements.

10. The stent as claimed in any one of claims 1 to 9, wherein the end points of said deformable bridges directly interconnected by straight longitudinal struts are all aligned in parallel to said central axis.

11. The stent as claimed in any one of claims 1 to 10, wherein said longitudinally deformable bridges are configured as serpentine links.

12. The stent as claimed in any one of claims 1 to 11, wherein
at least part of said longitudinally deformable bridges have the form of a Greek letter Ω (omega), the plane of symmetry of which is perpendicular to the central axis of the stent and which is connected at each side to a straight longitudinal strut.
